(19) Europäisches Patentamt
European Patent Office
Office européen des brevets

(11) Veröffentlichungsnummer: **0 025 113**
**B1**

(12) # EUROPÄISCHE PATENTSCHRIFT

(45) Veröffentlichungstag der Patentschrift: **20.07.83**

(51) Int. Cl.³: **C 07 D 285/00**, A 01 N 43/72 // C07C127/26, C07C143/833

(21) Anmeldenummer: **80104519.6**

(22) Anmeldetag: **31.07.80**

(54) **5.6-Dihydro-1.2.4.6-thiatriazin(5)on-1.1-dioxide, Verfahren zu ihrer Herstellung und diese enthaltende Herbizide.**

(30) Priorität: **22.08.79 DE 2933889**

(43) Veröffentlichungstag der Anmeldung: **18.03.81 Patentblatt 81/11**

(45) Bekanntmachung des Hinweises auf die Patenterteilung: **20.07.83 Patentblatt 83/29**

(84) Benannte Vertragsstaaten: **AT BE CH DE FR GB IT LI NL SE**

(56) Entgegenhaltungen:

DE-A-1 946 262
DE-A-2 026 625
DE-A-2 508 832

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Hamprecht, Gerhard, Dr., Rote-Turm-Strasse 28, D-6940 Weinheim (DE)**
Erfinder: **Acker, Rolf-Dieter, Dr., Tuchbleiche 8, D-6906 Leimen (DE)**
Erfinder: **Wuerzer, Bruno, Dr., Ruedigerstrasse 13, D-6701 Otterstadt (DE)**

EP 0 025 113 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 5,6-Dihydro-1,2,4,6-thiatriazin(5)on-1,1-dioxide, Verfahren zu ihrer Herstellung und diese enthaltende Herbizide

Die vorliegende Erfindung betrifft neue 5,6-Dihydro-1,2,4,6-thiatriazin(5)on-1,1-dioxide, Verfahren zu ihrer Herstellung, Herbizide, die diese Verbindungen als Wirkstoffe enthalten, sowie Verfahren zur Bekämpfung unerwünschten Pflanzenwuchses mit diesen Verbindungen.

Substituierte 5,6-Dihydro-1,2,4,6-thiatriazin(5)-on-1,1-dioxide mit dem Methylthiorest in 3-Stellung (DE-A-2 508 832) oder mit dem Methylrest in 4-Stellung (DE-A-1 946 262) und ihre herbizide Wirkung sind bekannt. Demgegenüber haben die neuen Wirkstoffe überraschend eine bessere herbizide Wirkung.

Es wurde nun gefunden, dass 5,6-Dihydro-1,2,4,6-thiatriazin(5)on-1,1-dioxidderivate der Formel

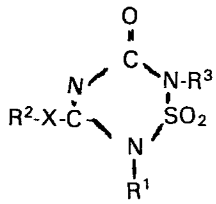

I

in der

$R^1$ Wasserstoff, ein Metallatom oder einen gegebenenfalls durch Alkyl ($C_1$-$C_{13}$) substituierten Ammoniumrest,

$R^2$ einen gesättigten oder ungesättigten geradkettigen aliphatischen Rest mit bis zu 10 Kohlenstoffatomen, einen cycloaliphatischen Rest mit 3 bis 7 Kohlenstoffatomen, einen verzweigten gesättigten oder ungesättigten aliphatischen Rest mit 3 bis 10 Kohlenstoffatomen, einen halogen-, methoxy- oder methylmercapto-substituierten aliphatischen Rest mit 2 bis 10 Kohlenstoffatomen, einen cyclohexoxy-substituierten aliphatischen Rest mit 4 bis 10 Kohlenstoffatomen, einen gegebenenfalls halogen-substituierten Benzylrest oder einen gegebenenfalls halogen-, tertiärbutyl- oder isopropyl-substituierten Phenylrest mit 1 bis zu 10 Kohlenstoffatomen bedeutet,

$R^3$ Wasserstoff, einen geradkettigen aliphatischen Rest mit bis zu 10 Kohlenstoffatomen, einen cycloaliphatischen Rest mit 3 bis 7 Kohlenstoffatomen, einen verzweigten aliphatischen Rest mit 3 bis 10 Kohlenstoffatomen oder einen halogen- oder methoxysubstituierten Alkylrest mit 2 bis 10 Kohlenstoffatomen bedeutet und

X Sauerstoff bedeutet, einerseits bei bestimmte Nutzpflanzen überraschende selektive herbizide Eigenschaften vorweisen und andererseits sowohl bei einjährigen unerwünschten Pflanzen als auch bei mehrjährigen schwer bekämpfbaren unerwünschten Pflanzen eine beachtliche herbizide Wirkung haben.

$R^1$ bedeutet in der Formel I beispielsweise Natrium, Kalium, Ammonium, Dimethylammonium, Tridecylammonium, Trimethylammonium, Diisopropylammonium.

In der Formel I bedeutet $R^2$ und $R^3$ beispielsweise Methyl, Äthyl, Propyl, Isopropyl, Cyclopropyl, Butyl, Isobutyl, sec-Butyl, tert-Butyl, Pentyl, Cyclopentyl, Hexyl, Cyclohexyl, 3-Pentyl, 1,2-Dimethylpropyl, 1,3-Dimethylbutyl, 2-Chloräthyl, 2-Chlorpropyl, 3-Chlorproyl, 2-Chlorisopropyl, 1-Chlormethylpropyl, 1-Äthyl-2-methylpropyl, 1,2,2-Trimethylpropyl, 1,2-Dimethylhexyl, 1-Cyclohexyläthyl, 2-Chlorbutyl-3, 2-Chlor-2-methypropyl, 2-Fluorbutyl-3, 2-Fluor-2-methylpropyl, 2-Fluorisopropyl, tert-Amyl, Chlor-tert-butyl, 2,2,2-Trifluoräthyl, Methoxyäthyl, 3-Methoxypropyl, Methoxyisopropyl, 3-Methoxybutyl, 1-Methoxy-butyl-2, Methoxy-tert-butyl, 2-Methoxy-butyl, 4-Methoxy-butyl.

Darüber hinaus kann $R^2$ beispielsweise Allyl, Methallyl, Crotyl, 2-Äthyl-hexen-2-yl-1, Hexen-5-yl-1, 2-Methyl-buten-2-yl-1, 2-Methyl-buten-1-yl-3, Butin-1-yl-3, Butin-2-yl-1, Buten-1-yl-3, Propargyl, 2-Methyl-buten-1-yl-4, 2-Methyl-buten-2-yl-4, 3-Methyl-buten-1-yl-3, Methylmercapto-äthyl, 3-Methylmercapto-propyl, 3-Methylmercapto-butyl, 1-Methylmercapto-butyl-2, Methylmercapto-tert-butyl, 2-Methylmercapto-butyl, Cyclohexoxy-äthyl, Benzyl, 2,6-Dichlorbenzyl, 2-Chlor-6-fluorbenzyl, 2,6-Difluorbenzyl, Phenyl, 4-Chlorphenyl, 2,4-Dichlorphenyl, 4-Isopropylphenyl, oder 4-tert-Butylphenyl bedeuten.

Die Bezeichnung «Halogen» bedeutet Fluor, Chlor, Brom oder Jod.

Weiterhin wurde gefunden, dass man 1,2,4,6-Thiatriazin(5)on-1,1-dioxidderivate gemäss Formel I, wobei X für Sauerstoff steht, erhält, wenn man N-Carboalkoxy-O-alkylisoharnstoffe der Formel

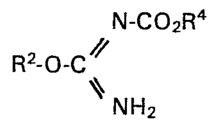

III

in der $R^2$ die vorgenannte Bedeutung besitzt und $R^4$ einen Alkylrest mit 1 bis 4 Kohlenstoffatomen bedeutet, mit einem Aminosulfonylhalogenid der Formel

$$R^3\text{-}NHSO_2Y \qquad IV$$

in der $R^3$ die vorgenannte Bedeutung besitzt und Y für Fluor oder Chlor steht, gegebenenfalls in Gegenwart eines Säureakzeptors und eines inerten organischen Lösungsmittels bei einer Temperatur von −20 bis +80°C zu einem Sulfondiamid der Formel

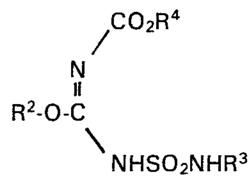

II

in der $R^2$, $R^3$ und $R^4$ die vorgenannten Bedeutungen besitzen, drucklos oder unter Druck, kontinuierlich oder diskontinuierlich umsetzt und dieses — gegebenenfalls ohne Isolierung — in Gegenwart einer basischen Verbindung bei einer Temperatur von 0 bis 100°C cyclisiert.

N-Carboalkoxy-O-alkylisoharnstoffe der Formel III sind bekannt oder lassen sich nach bekannten Methoden herstellen.

Verwendet man N-Carbomethoxy-O-methyliso-

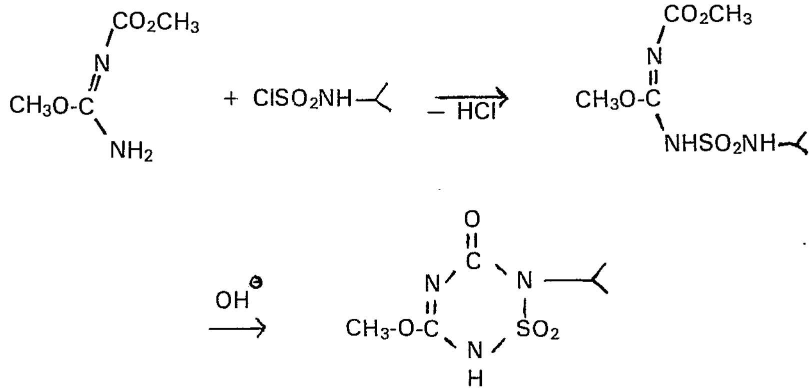


Man verwendet unter den Reaktionsbedingungen inerte organische Lösungsmittel. Als Lösungsmittel kommen z.B. in Frage: Halogenkohlenwasserstoffe, insbesondere Chlorkohlenwasserstoffe, z.B. Tetrachloräthylen, 1,1,2,2- oder 1,1,1,2-Tetrachloräthan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichloräthan, Trichloräthylen, Pentachloräthan, o-, m-, p-Difluorbenzol, 1,2-Dichloräthan, 1,1-Dichloräthan, 1,2-cis-Dichloräthylen, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol; Äther, z.B. Äthylpropyläther, Methyl-tert-butyläther, n-Butyläthyläther, Di-n-butyläther, Diisobutyläther, Diisoamyläther, Diisopropyläther, Anisol, Phenetol, Cyclohexylmethyläther, Diäthyläther, Äthylenglykoldimethyläther, Tetrahydrofuran, Dioxan, Thioanisol, β,β-Dichlordiäthyläther; Nitrokohlenwasserstoffe wie Nitromethan, Nitroäthan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalls von 70 bis 190°C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan; Ester, z.B. Äthylacetat, Acetessigester, Isobutylacetat; Amide, z.B. Formamid, Methylformamid, Dimethylformamid; Ketone, z.B. Aceton, Methyläthylketon; und entsprechende Gemische. Zweckmässig verwendet man das Lösungsmittel in einer Menge von 100 bis 2.000 Gew.-%, vorzugsweise von 200 bis 700 Gew.-%, bezogen auf Ausgangsstoff III.

Als Säureakzeptoren können alle üblichen Säurebindemittel verwendet werden. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Es kommen z.B. als basische Verbindungen in Frage: Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Triäthylamin, Tripropylamin, Triisopropylamin, Tributylamin, Triisobutylamin, Tri-sec-butylamin, Tri-tert-butylamin, Tribenzylamin, Tricyclohexylamin, Triamylamin, Trihexylamin, N,N-Dimethylanilin, N,N-Diäthylanilin, N,N-Dipropylanilin, N,N-Dimethyltoluidin, N,N-Diäthyltoluidin, N,N-Dipropyltoluidin, N,N-Dimethyl-p-aminopyridin, N,N-Diäthyl-p-aminopyridin, N,N-Dipropyl-p-aminopyridin, N-Methylpyrrolidon, N-Äthylpyrrolidon, N-Methylpiperidin, N-Äthylpiperidin, N-Methylpyrrolidin, N-Äthylpyrrolidin, N-Methylimidazol, N-Äthylimidazol, N-Methylpyrrol, N-Äthylpyrrol, N-Methylmorpholin, N-Äthylmorpholin, N-Methylhexamethylenimin, N-Äthylhexamethylenimin, Pyridin, Chinolin, α-Picolin, β-Picolin, α-Picolin, Isochinolin, Pyrimidin, Acridin, N,N,N',N'-Tetramethyläthylendiamin, N,N,N'N'-Tetraäthyläthylendiamin, Chinoxalin, Chinazolin, N-Propyldiisopropylamin, N,N-Dimethylcyclohexylamin, 2,6-Lutidin, 2,4-Lutidin, Trifurfurylamin, Triäthylendiamin.

Als Cyclisierungsagentien kommen z.B. die vorgenannten anorganischen Säurebindemittel in Frage, ferner z.B. Natriumpropionat, Natriumbutyrat, Natriumisobutyrat, Kaliumformiat, Kaliumacetat, Kaliumbutyrat, Kaliumisobutyrat, Natriummethylat, Natriumäthylat, Natriumpropylat, Natriumisopropylat, Natriumbutylat, Natriumisobutylat, Natrium-sec-butylat, Natrium-tert-butylat, Natriumäthylenglykolat, Natriumpropylen-(1,2)-glykolat, Natriumpropylen-(1,3)-glykolat, Natriumdiäthylenglykolat, Natriumtriäthylenglykolat, Natriumdipropylen-(1,2)-glykolat, Kaliummethylat, Kaliumäthylat, Kalium-n-propylat, Kaliumisopropylat, Kalium-n-butylat, Kalium-isobutylat, Kalium-sec-butylat, Kalium-tert-butylat, Kaliumäthylenglykolat, Kaliumpropylen-(1,2)-glykolat, Kaliumpropylen-(1,3)-glykolat, Kaliumdiäthylenglykolat, Kaliumtriäthylenglykolat, Kaliumdipropylen-(1,2)-glykolat.

Das Säurebindemittel wird zweckmässig in äquivalenten Mengen oder in einem Überschuss von bis zu 20%, bezogen auf Aminosulfonylhalogenid der Formel IV, eingesetzt.

Die Cyclisierung wird unter Zusatz der 1- bis 2,5fachen molaren Menge an basischem Cyclisierungsmittel bezogen auf Sulfondiamid der Formel II durchgeführt.

Die Ausgangsstoffe der Formeln III und IV werden in ungefähr stöchiometrischem Verhältnis eingesetzt, d.h. in einem Unter- bzw. Überschuss von bis zu 20% Ausgangsstoff der Formel IV bezogen auf Ausgangsstoff der Formel III.

Zweckmässig wird das Verfahren so durchgeführt, dass man über zwei Zuführungen das Aminosulfonylhalogenid der Formel IV und die äquivalente Menge an Säureakzeptor bei einer Temperatur zwischen −20 bis 80°C, vorzugsweise 0 bis 40°C zu einer ungefähr äquivalenten Menge an N-Carboalkyl-O-alkylisoharnstoff der Formel III in einem inerten organischen Lösungsmittel zulaufen lässt.
Man kann jedoch auch ein Gemisch des Ausgangsstoffs III und des Säureakzeptors in einem inerten organischen Lösungsmittel vorlegen und dann bei −20 bis 80°C, vorzugsweise bei 0 bis 40°C das Aminosulfonylhalogenid der Formel IV zulaufen lassen. Zur Beendigung der Umsetzung rührt man noch 0,5 bis 8 Stunden bei −20 bis 80°C, vorzugsweise bei 0 bis 40°C nach.

Das Reaktionsgemisch wird dann gegebenenfalls eingeengt bzw. im Falle mit Wasser nicht mischbarer Lösungsmittel direkt mit verd. Salzsäure und mit Wasser zur Entfernung der Hydrochloride extrahiert, wobei ein Sulfondiamid der Formel II erhalten wird.

Dieses kann man in wässrigem Medium in Gegenwart der 1- bis 2,5fachen Menge Base oder auch in organischem Medium in Gegenwart der 1- bis 2,5-fachen Menge Alkoholat zu dem gewünschten 1,2,-4,6-Thiatriazin(5)on-1,1-dioxid-salz cyclisieren. Zur Aufarbeitung säuert man danach an und saugt den ausgefallenen Niederschlag, gegebenenfalls nach weiterem Einengen ab. Die gewünschten Endstoffe fallen hierbei in reiner Form an, gegebenenfalls können sie durch Umkristallisieren oder Chromatographie gereinigt werden.

Im Falle von $R^1$ = Wasserstoff liegen sie nach ihren spektroskopischen Daten in der durch die Formel I wiedergegebenen Strukturform vor. Je nach Lösungsmittel können dabei jedoch auch gewisse Anteile der tautomeren Form I a auftreten, die als im Gleichgewicht befindliche Verbindungen durch die Formel I mitbeansprucht und umfasst werden.

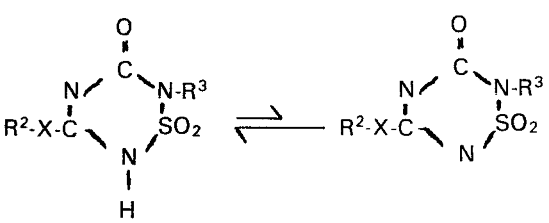

I I a

Die zur Herstellung der Ausgangsmaterialien III erforderlichen substituierten Isoharnstoffe sind bekannt oder lassen sich nach bekannten Methoden z.B. Beilstein, IV. Auflage, 3. Ergänzungswerk, Bd. III, S. 146-148 herstellen. Ihre Umsetzung mit Acylhalogeniden sind ebenfalls bekannt, z.B. S. Basterfield und M.S. Whelen, J. Am. chem. Soc. *49*, 3177 (1927); ferner US-PS 4 014 924. Nach diesen Methoden können beispielsweise folgende substituierte N-Carbomethoxy-O-alkyl-isoharnstoffe hergestellt werden:

| 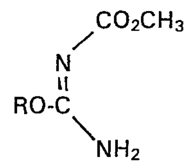 R | Fp (°C)/$n_D^{25}$ |
|---|---|
| $CH_3$ | 43-46 |
| $C_2H_5$ | 61-63 |
| n-$C_3H_7$ | 48-52 |
| i-$C_3H_7$ | 45-50 |
| n-$C_4H_9$ | 1.4657 |
| i-$C_4H_9$ | 37 |
| sec-$C_4H_9$ | 1.4622 |
| 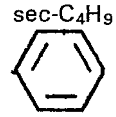 -$CH_2$ | 68-72 |
| 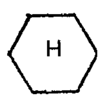 | 97-100 |

Die neuen Verbindungen können beispielsweise nach folgendem Verfahren hergestellt werden:

*Beispiel 1*

198 Teile (Gew.-Teile) Methylaminosulfonylchlorid u. 162 Teile Triäthylamin wurden gleichzeitig über 2 Zuführungen bei 25 bis 30°C unter Rühren in eine Mischung von 202 Teilen N-Carbomethoxy-O-methylisoharnstoff in 1570 Teilen Acetonitril eingeführt. Nach 3 Stunden Rühren bei 25°C wurde vom ausgefallenen Hydrochlorid abgesaugt und das Filtrat im Vakuum eingeengt. Der Rückstand wurde in 1500 Teilen 1,2-Dichloräthan gelöst, einmal mit Wasser und zweimal mit 0,5 normaler Salzsäure extrahiert. Nach dem Trocknen über Magnesiumsulfat und Einengen im Vakuum wurden 257 Teile N-Carbomethoxy-N'-methylsulfamoyl-O-methylharnstoff mit $n_D^{25}$ 1,4851 erhalten.

Hiervon wurden 96 Teile in 235 Teilen Methanol (absolut trocken) gelöst, mit 153,5 Teilen Natriummethylat (30 Gew.-%) versetzt und 3 Stunden unter Rückfluss gerührt. Nach dem Einengen im Vakuum wurde der Rückstand in Wasser gelöst, einmal mit Äther extrahiert und mit verd. Schwefelsäure angesäuert. Nach dem Absaugen, Waschen mit Wasser und Trocknen wurden 68 Teile (= 82,5% der Theorie) 6-Methyl-3-methoxy-5,6-dihydro-1,2,4,6-thiatriazin(5)on-1,1-dioxid mit Fp. 198 bis 202°C erhalten. (Wirkstoff Nr. 1)

*Beispiel 2*

59,6 Teile Cyclohexylaminosulfonylchlorid und 26,9 Teile Pyridin wurden über zwei Zuführungen bei

15 bis 20°C unter Rühren in eine Lösung von 39,6 Teilen N-Carbomethoxy-O-methylisoharnstoff in 300 Teilen Essigester eingeführt. Nach 4 Stunden Rühren bei 25°C wurde das Reaktionsgemisch je einmal mit Wasser und mit 0,5 normaler Salzsäure extrahiert, getrocknet und dann im Vakuum eingeengt. Hierbei wurden 79 Teile N-Carbomethoxy--N'-cyclohexylsulfamoyl-O-methylisoharnstoff mit $n_D^{25}$ 1,4970 erhalten. Nach dem Anreiben mit wenig Äther kristallisierte die Verbindung mit Fp. 84 bis 86°C. 15 Teile n-Carbomethoxy-N'-cyclohexylsulfamoyl-O-methyisoharnstoff wurden in einer Mischung von 9 Teilen Natronlauge (50 Gew.-%) und 20 Teilen Wasser gelöst und 4 Minuten bei 55 bis 60°C gerührt. Nach dem Abkühlen wurde die Reaktionslösung einmal mit Äther extrahiert und dann in eine Mischung von 9,5 Teilen konz. Salzsäure in 10 Teilen Wasser eingerührt. Nach dem Absaugen, Waschen mit Wasser und Trocknen wurden 9 Teile 6-Cyclohexyl-3-methoxy-5.6-dihydro-1,2,4,6-thiatriazin(5)on-1,1-dioxid mit Fp. 173 bis 177°C erhalten. (Wirkstoff Nr. 3)

*Beispiel 3*

95 Teile Isopropylaminosulfonylchlorid wurden bei 10 bis 15°C innerhalb 25 Minuten in eine Mischung von 96 Teilen n-Carbomethoxy-O-isopropylisoharnstoff und 73 Teilen Triäthylamin in 700 Teilen Tetrahydrofuran eingerührt. Nach einer Stunde Rühren bei 25°C wurde das Reaktionsgemisch je einmal mit Wasser und mit 0,5 normaler Salzsäure extrahiert, getrocknet und im Vakuum eingeengt. Dabei wurden 130 Teile N-Carbomethoxy-N'-isopropylsulfamoyl-O-isopropylisoharnstoff mit Fp. 62 bis 64°C erhalten. Hiervon wurden 33,7 Teile mit 17,6 Teilen Natronlauge (50 Gew.-%) in 30 Teilen Wasser während 5 Minuten bei 55 bis 60°C cyclisiert. Nach dem Extrahieren mit Äther, Ansäuern, Waschen mit Wasser und Trocknen wurden 22 Teile 6-Isopropyl--3-isopropoxy-5,6-dihydro-1,2,4,6-thiatriazin(5)-on-1,1-dioxid mit Fp. 164 bis 167°C erhalten. (Wirkstoff Nr. 4)

*Beispiel 4*

12 Teile 6-Isopropyl-3-isopropoxy-5,6-dihydro--1,2,4,6-thiatriazin(5)on-1,1-dioxid wurden in einer Mischung von 10,4 Teilen Natriummethylat (30 Gew.-%) und 64 Teilen Methanol bei 25°C gelöst. Nach dem Einengen wurden 13,8 Teile 6-Isopropyl--3-isopropoxy-5,6-dihydro-1,2,4,6-thiatriazin(5)-on-1,1-dioxid-2-natriumsalz mit Fp. 123°C Zersetzung erhalten. (Wirkstoff Nr. 5)

*Beispiel 5*

140 Teile n-Carboxymethyl-N'-methylsulfamoyl--O-methylharnstoff wurden in einer Mischung von 79,5 Teilen Natriumcarbonat in 450 Teilen Wasser und 31 Volumenteilen 2 n Natronlauge 10 Minuten bei 45°C gerührt. Das Reaktionsgemisch wurde abgekühlt, mit Äther extrahiert und langsam in eine Mischung von 78 Teilen konz. Schwefelsäure in 150 Teilen Eiswasser eingerührt. Nach dem Absaugen, Waschen mit Wasser und Trocknen wurden 81 Teile 6-Methyl-3-methoxy-5,6-dihydro-1,2,4,6-thiatriazin(5)on-1,1-dioxid (68% der Theorie) mit Fp. 195 bis 199°C erhalten. (Wirkstoff Nr. 1)

Weitere Beispiele für neue Verbindungen sind in der nachfolgenden Tabelle aufgeführt:

Tabelle

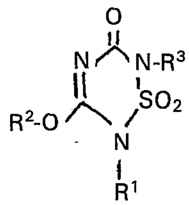

| Wirkstoff Nr. | $R^1$ | $R^2$ | $R^3$ | Fp (°C) |
|---|---|---|---|---|
| 7 | Na | $CH_3$ | $CH_3$ | 246-252 |
| 9 | H | $CH_3$ | $C_2H_5$ | 167-170 |
| 10 | Na | $CH_3$ | $C_2H_5$ | 143 Zers. |
| 12 | H | $CH_3$ | $n-C_3H_7$ | 137-140 |
| 13 | Na | $CH_3$ | $n-C_3H_7$ | 214 Zers. |
| 14 | H | $CH_3$ | $i-C_3H_7$ | 146-150 |
| 15 | Na | $CH_3$ | $i-C_3H_7$ | 265 Zers. |
| 17 | H | $CH_3$ | $n-C_4H_9$ | 128-132 |
| 18 | Na | $CH_3$ | $n-C_4H_9$ | 219 Zers. |
| 25 | Na | $CH_3$ | 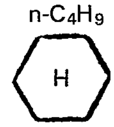 | 216-220 |
| 29 | H | $CH_3$ | $CH_2-CH_2-O-CH_3$ | 180-182 |
| 30 | Na | $CH_3$ | $-CH_2-CH_2-O-CH_3$ | 111-115 |
| 32 | H | $C_2H_5$ | $CH_3$ | 168-171 |
| 37 | H | $C_2H_5$ | $n-C_3H_7$ | 122-125 |
| 39 | H | $C_2H_5$ | $i-C_3H_7$ | 154-158 |
| 40 | Na | $C_2H_5$ | $i-C_3H_7$ | 116 Zers. |
| 49 | Na | $n-C_3H_7$ | $CH_3$ | 155-160 |
| 50 | H | $n-C_3H_7$ | $C_2H_5$ | 117-123 |
| 51 | H | $n-C_3H_7$ | $i-C_3H_7$ | 162-166 |
| 52 | Na | $n-C_3H_7$ | $i-C_3H_7$ | 152 |
| 61 | H | $i-C_3H_7$ | $CH_3$ | 174-176 |
| 62 | Na | $i-C_3H_7$ | $CH_3$ | 305 Zers. |
| 64 | H | $i-C_3H_7$ | $n-C_3H_7$ | 107-111 |
| 68 | H | $n-C_4H_9$ | $CH_3$ | 112-116 |
| 69 | Na | $n-C_4H_9$ | $CH_3$ | 156-160 |
| 70 | H | $n-C_4H_9$ | $C_2H_5$ | 117-123 |
| 74 | H | $i-C_4H_9$ | $CH_3$ | 138 Zers. |
| 79 | H | $i-C_4H_9$ | $i-C_3H_7$ | 131-135 |
| 80 | Na | $i-C_4H_9$ | $i-C_3H_7$ | 163 Zers. |
| 86 | H | $sec-C_4H_9$ | $CH_3$ | 114-118 |
| 87 | Na | $sec-C_4H_9$ | $CH_3$ | 172 Zers. |
| 90 | H | $sec-C_4H_9$ | $i-C_3H_7$ | 132-135 |
| 91 | Na | $sec-C_4H_9$ | $i-C_3H_7$ | 178 Zers. |
| | | 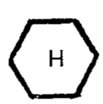 | $i-C_3H_7$ | 159-163 |
| 124 | H | 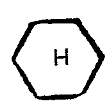 | $CH_3$ | 172-174 |

Die neuen Substanzen können in die üblichen Formulierungen übergeführt werden, wie Lösungen, Emulsionen, Suspensionen, Stäube, Pulver, Pasten und Granulate. Die Anwendungsformen richten sich

ganz nach den Verwendungszwecken; sie sollen in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen - Fettalkohol - Ether, Alkylsulfonate und Arylsulfonate) und Dispergiermittel, wie Lignin, Sulfitablaugen und Methylcellulose.

Die herbiziden Mittel enthalten im allgemeinen zwischen 0,1 und 95 Gew.-% Wirkstoff, vorzugsweise zwischen 0,5 und 90.

Die Aufwandmengen liegen je nach Zusammensetzung und Wachstumsstadien der Unkrautflora zwischen 0,1 und 15, vorzugsweise jedoch zwischen 0,2 und 5 kg Wirkstoff pro Hektar, wobei für eine totale Pflanzenvernichtung die höheren Aufwandmengen zu verwenden sind.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Giessen.

Beispiele für solche Zubereitungen sind:

I. Man vermischt 90 Gewichtsteile der Verbindung des Beispiels 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung des Beispiels 2 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingiessen und feines Verteilen der Lösung in 100.000 Gewichtsteilen Wasser erhält man eine wässrige Dispersion, die 0,02 Gew.-% des Wirkstoffs enthält.

III. 20 Gewichtsteile der Verbindung des Beispiels 3 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingiessen und feines Verteilen der Lösung in 100.000 Gewichtsteilen Wasser erhält man eine wässrige Dispersion, die 0,02 Gew.-% des Wirkstoffs enthält.

IV. 20 Gewichtsteile der Verbindung des Beispiels 1 werden den mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselgel gut vermischt und in einer Hammermühle vermahlen. Durch feines Verteilen der Mischung in 20.000 Gewichtsteilen Wasser erhält man eine Spritzbrühe, die 0,1 Gew.-% des Wirkstoffs enthält.

V. 30 Gewichtsteile der Verbindung des Beispiels 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VI. 40 Gewichtsteile der Verbindung des Beispiels 3 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wässrige Dispersion. Durch Verdünnen mit 100.000 Gewichtsteilen Wasser erhält man eine wässrige Dispersion, die 0,04 Gew.-% Wirkstoff enthält.

VII. 20 Teile der Verbindung des Beispiels 1 werden mit 2 Teilen Calciumsalz der Dodecylbenzolsulfonsäure, 8 Teilen Fettalkoholpolyglykolether, 2 Teilen Natriumsalz eines Phenolsulfonsäureharnstoffformaldehyd-Kondensates und 68 Teilen eines paraffinischen Mineralöls innig vermischt. Man erhält eine stabile ölige Dispersion.

Die neuen 5,6-Dihydro-1,2,4,6-thiatriazin(5)on-1,1-dioxide können unter sich und mit zahlreichen Vertretern anderer herbizider oder wachstumsregulierender Wirkstoffgruppen gemischt und gemeinsam ausgebracht werden. Beispielsweise kommen als Mischungskomponente Diazine, Benzothiadiazinone, 2,6-Dinitroaniline, N-Phenyl-carbamate, Biscarbamate, Thiolcarbamate, Halogencarbonsäuren, Triazine, Amide, Harnstoffe, Diphenyläther, Triazinone, Uracile, Benzofuranderivate und andere in Betracht. Solche Kombinationen dienen zur Verbreiterung des Wirkungsspektrums und erzielen zuweilen synergistische Effekte.

Ausserdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden.

Zur Aktivierung der herbiziden Wirkung können Netz- und Haftmittel sowie nicht-phytotoxische Öle und Ölkonzentrate zugesetzt werden.

Der Einfluss verschiedener Vertreter der erfindungsgemässen Verbindungen auf das Wachstum von unerwünschten und erwünschten Pflanzen wird in nachfolgenden Gewächshausversuchen demonstriert:

Die Samen der Testpflanzen entsprechend Tabelle 1 wurden nach Arten getrennt flach eingesät. Bei Cyperus esculentus pflanzte man angekeimte Knollen ein und Mentha piperita wurde ebenfalls vegetativ durch Stecklinge vermehrt. Letztere wurde daher auch nur zur Nachauflaufbehandlung herangezogen

Unmittelbar nach der Einsaat erfolgte bei Vorauflaufbehandlung das Aufbringen der Wirkstoffe auf die Erdoberfläche. Die Wirkstoffe wurden hierbei in Wasser als Verteilungsmittel suspendiert oder emulgiert und mittels fein verteilender Düsen gespritzt. Nach dem Aufbringen der Mittel wurden die Gefässe leicht beregnet, um Keimung und Wachstum in Gang zu bringen und auch gleichzeitig die Wirkstoffe zu aktivieren. Danach deckte man die Gefässe mit durchsichtigen Plastikhauben ab, bis die Pflanzen angewachsen waren. Diese Abdeckung bewirkte ein gleichmässiges Keimen der Testpflanzen, sofern dies nicht durch die Wirkstoffe beeinträchtigt wurde, und verhinderte das Verdampfen leicht flüchtiger Substanzen.

Zum Zwecke der Nachauflaufbehandlung zog man die Pflanzen je nach Wuchsform in den Versuchsgefässen erst bis zu einer Höhe von 3 bis 10 cm an und behandelte sie danach. Eine Abdeckung unterblieb. Die Aufstellung der Versuche erfolgte im Gewächshaus, wobei für wärmeliebende Arten wärmere Bereiche (25 bis 40°C) und für solche gemässigter Klimate 15 bis 30°C bevorzugt wurden.

Die Versuchsperiode erstreckte sich über 3 bis 5 Wochen. Während dieser Zeit wurden die Pflanzen gepflegt und ihre Reaktion auf die einzelnen Behandlungen wurde ausgewertet. Die folgenden Tabellen enthalten die Prüfsubstanzen, die jeweiligen Dosierungen in kg/ha Aktivsubstanz und die Testpflanzenarten. Bewertet wird nach einer Skala von 0 bis 100. Dabei bedeutet 0 keine Schädigung oder normaler Ablauf und 100 kein Aufgang der Pflanzen bzw. völlige Zerstörung zumindest der oberirdischen Sprossteile.

Die beigefügten Tabellen zeigen die selektive herbizide Wirkung der neuen Verbindungen. Diese richtet sich in erster Linie gegen Cyperaceen und breitblättrige unerwünschte Pflanzen, wobei auch eine beachtliche Wirkung auf mehrjährige Arten unter den Versuchspflanzen festzustellen ist. Dabei sind die Mittel neben Gramineenkulturpflanzen überraschenderweise noch für einige breitblättrige Kulturpflanzen verträglich. Sie werden im Vorauflauf- und Nachauflaufverfahren eingesetzt. Eine besondere Ausbringungstechnik besteht darin, dass die Wirkstoffe mit Hilfe der Spritzgeräte so gespritzt werden, dass die Blätter empfindlicher Kulturpflanzen nach Möglichkeit nicht getroffen werden, während die Wirkstoffe auf die darunterliegende Bodenfläche oder dort wachsende unerwünschte Pflanzen gelangen (post-directed, lay-by).

In Anbetracht der Vielseitigkeit der Applikationsmethoden können die Mittel oder diese enthaltende Mischungen noch in einer weiteren grossen Zahl von Kulturen zur Beseitigung unerwünschten Pflanzenwuchses eingesetzt werden.

Im einzelnen seien folgende Nutzpflanzen genannt:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuss | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fodder beets |
| Beta vulgaris spp. esculenta | Rote Rübe | table beets, red beets |
| Brassica napus var. napus | Raps | rape seed |
| Brassica napus var. napobrassica | Kohlrübe | |
| Brassica napus var. rapa | Weisse Rübe | turnips |
| Brassica rapa var. silvestris | Rüben | |
| Camellia sinensis | Teestrauch | tea plants |
| Carthamus tinctorius | Saflor - Färberdistel | safflower |
| Carya illinoinensis | Pekannussbaum | pecan trees |
| Citrus limon | Zitrone | lemon |
| Citrus maxima | Pempelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagras | Bermudagrass in turfs and lawns |
| Daucus carota | Möhre | carrots |
| Elaeis guineensis | Ölpalme | oil palms |
| Fragaria vesca | Erdbeere | strawberries |
| Glycine max | Sojabohne | soybeans |
| Gossypium hirsutum (Gossypium arboreum, Gossypium herbaceum, Gossypium vitifolium) | Baumwolle | cotton |
| Helianthus annuus | Sonnenblume | sunflowers |
| Helianthus tuberosus | Topinambur | |

| Botanischer Name | Deutscher Name | Englischer Name |
|---|---|---|
| Hevea brasiliensis | Parakautschukbaum | rubber plants |
| Hordeum vulgare | Gerste | barley |
| Humulus lupulus | Hopfen | hop |
| Ipomoea batatas | Süsskartoffeln | sweet potato |
| Juglans regia | Walnussbaum | walnut trees |
| Lactua sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |
| Manihot esculenta | Maniok | cassava |
| Medicago sativa | Luzerne | alfalfa (lucerne) |
| Mentha piperita | Pfefferminze | peppermint |
| Musa spp. | Obst- und Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beans, dry beans |
| Pennisetum glaucum | Perl- oder Rohkohlbenhirse | |
| Petroselinum crispum spp. tuberosum | Wurzelpetersilie | parsley |
| Picea abies | Rotfichte | Norway spruce |
| Abies alba | Weisstanne | fire |
| Pinus spp. | Kiefer | pine trees |
| Pisum sativum | Gartenerbse | English peas |
| Prunus avium | Süsskirsche | cherry trees |
| Prunus domestica | Pflaume | plum trees |
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | Sesame |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preisselbeere | cranberry |
| Vicia faba | Pferdebohnen | tick peans |
| Vigna sinensis | Kuhbohne | cow peas |
| Vitis vinifera | Weinrebe | grapes |
| Zea mays | Mais | Indian corn, sweet corn, maize |

Tabelle 1

| Botanischer Name | Abkürzung in | Deutsche Bezeichnung | Eglische Bezeichnung |
|---|---|---|---|
| Arachys hypogaea | | Erdnuss | peanuts (groundnuts) |
| Centaurea cyanus | | Kornblume | cornflower |
| Chenopodium album | | Weisser Gänsefuss | lambsquarters (goosefoot) |
| Cucumis sativum | | Gurke | cucumber |
| Cyperus esculentus | Cyperus escul. | Erdmantel | yellow nutsedge |

| Botanischer Name | | Deutscher Name | Englischer Name |
|---|---|---|---|
| Datura stramonium | | gemeiner Stechapfel | Jimsonweed |
| Euphorbia geniculata | | Südamerik. Wolfsmilchart | Southamericana member of the spurge family |
| Gossypium hirsutum | | Baumwolle | cotton |
| Mentha piperita | Mentha pip. | Pfefferminze | peppermint |
| Nicandra physaloides | | Giftbeere | apple of Peru |
| Sinapis alba | | Weisser Senf | white mustard |
| Sorghum bicolor | | Mohrenhirse (Kulturhirse) | sorghum |
| Solanum nigrum | | Schwarzer Nachtschatten | black nightshade |
| Asclepias spp. | Asclep. spp. | Seidenpflanze | milkweed |
| Chenopodium album | Chenop. alb. | Weisser Gänsefuss | lambsquarters |
| Cyperus iria | Cyper. iria | — | annual sedge species |
| Datura stramonium | Datura stram. | gemeiner Stechapfel | jimsonweed |
| Matricaria spp. | Matric. spp. | Kamillenarten | chamomile |

Tabelle 2

Selektive Beseitigung von unerwünschten Pflanzen in Gurken bei Nachauflaufanwendung im Gewächshaus

Testpflanzen Wirkstoffe

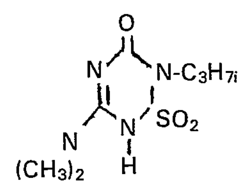

| Testpflanzen | Nr. 12 | Nr. 39 | bekannt |
|---|---|---|---|
| | Schädigung % bei 1,0 kg/ha | | |
| Cucumis sativum | 5 | 0 | 60 |
| Centaurea cyanus | 90 | 95 | 80 |
| Nicandra physaloides | 80 | — | 100 |
| Sinapis alba | 69 | 94 | 99 |
| Solanum nigrum | 95 | 100 | 100 |

Tabelle 3

Bekämpfung von breitblättrigen Unkräutern in Erdnüssen bei Nachauflaufanwendung im Gewächshaus

Testpflanzen Wirkstoffe

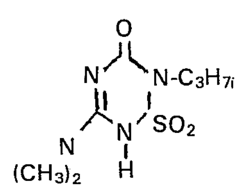

| Testpflanzen | Nr. 61 | bekannt |
|---|---|---|
| | Schädigung % bei 2,0 kg/ha | |
| Arachys hypogaea | 5 | 30 |
| Centaurea cyanus | 95 | 90 |
| Chenopodium album | 98 | 70 |
| Euphorbia geniculata | 95 | 45 |
| Nicandra physaloides | 100 | 100 |

Tabelle 4

Weiteres Beispiel zur selektiven Bekämpfung von Unkräutern in Kulturen bei Nachauflaufanwendung im Gewächshaus

Testpflanzen Wirkstoffe

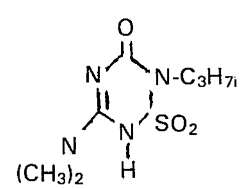

| Testpflanzen | Nr. 91 | bekannt |
|---|---|---|
| | Schädigung % bei 2,0 kg/ha | |
| Gossypium hirsutum | 10 | 45 |
| Sorghum bicolor | 0 | 60 |
| Centaurea cyanus | 90 | 90 |
| Datura stramonium | 100 | 100 |
| Nicandra physaloides | 100 | 100 |
| Sinapis alba | 100 | 100 |

Tabelle 5

Beispiel zur Bekämpfung perennierender Pflanzen bei Vor- und Nachauflaufanwendung im Gewächshaus

| Wirkstoff | kg/ha | Testpflanzen und Schädigung % Vorauflauf Cyperus escul. | Nachauflauf Cyperus escul. | Mentha pip. |
|---|---|---|---|---|
| 7 2,0 | 89 | 64 | 75 | |
| | 3,0 | – | 100 | 100 |
| 32 | 2,0 | 95 | 75 | 85 |

Tabelle 6

Selektive Bekämpfung von unerwünschten Pflanzen bei Nachauflaufanwendung im Gewächshaus

| Wirkstoff Nr. | kg/ha | Gossyp. hirs. | Oryza sat. | Tritic. aest. | Asclep. spp. | Chenop. alb | Cyper. iria | Datura stram. | Matric. spp. |
|---|---|---|---|---|---|---|---|---|---|
| | | | | Testpflanzen und Schädigung % | | | | | |
| 64 | 1,0 | 0 | 0 | 0 | 70 | 100 | 90 | 90 | 100 |

0 = keine Schädigung
100 = Pflanzen abgestorben

**Patentansprüche für die Vertragsstaaten:**
BE, CH, DE, FR, GB, IT, LI, NL, SE

1. 5,6-Dihydro-1,2,4,6-thiatriazin(5)on-1,1-dioxid der Formel

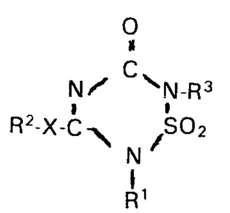

I

in der

$R^1$ Wasserstoff, ein Metallatom oder einen gegebenenfalls durch Alkyl ($C_1$-$C_{13}$) substituierten Ammoniumrest,

$R^2$ einen gesättigten oder ungesättigten geradkettigen aliphatischen Rest mit bis zu 10 Kohlenstoffatomen, einen cycloaliphatischen Rest mit 3 bis 7 Kohlenstoffatomen, einen verzweigten gesättigten oder ungesättigten aliphatischen Rest mit 3 bis 10 Kohlenstoffatomen, einen halogen-, methoxy- oder methylmercapto-substituierten aliphatischen Rest mit 2 bis 10 Kohlenstoffatomen, einen cyclohexoxy-substituierten aliphatischen Rest mit 4 bis 10 Kohlenstoffatomen, einen gegebenenfalls halogen-substituierten Benzylrest oder einen gegebenenfalls halogen-, tertiärbutyl- oder isopropyl-substituierten Phenylrest mit bis zu 10 Kohlenstoffatomen,

$R^3$ Wasserstoff, einen geradkettigen aliphatischen Rest mit bis zu 10 Kohlenstoffatomen, einen cycloaliphatischen Rest mit 3 bis 7 Kohlenstoffatomen, einen verzweigten aliphatischen Rest mit 3 bis 10 Kohlenstoffatomen oder einen halogen- oder methoxysubstituierten Alkylrest mit 2 bis 10 Kohlenstoffatomen und

X Sauerstoff bedeutet.

2. 5,6-Dihydro-1,2,4,6-thiatriazin(5)on-1,1-dioxid gemäss Anspruch 1, nämlich 6-Methyl-3-methoxy-5,6-dihydro-1,2,4,6-thiatriazin(5)on-1,1-dioxid, 6-Methyl-3-äthoxy-5,6-dihydro-1,2,4,6-thiatriazin(5)on-1,1-dioxid, 6-Äthyl-3-methoxy-5,6-dihydro-1,2,4,6-thiatriazin(5)on-1,1-dioxid od. 6-Äthyl-3-äthoxy-5,6-dihydro-1,2,4,6-thiatriazin-(5)on-1,1-dioxid.

3. Herbizid, enthaltend ein 5,6-Dihydro-1,2,4,6-thiatriazin(5)on-1,1-dioxid gemäss Anspruch 1.

4. Herbizid gemäss Anspruch 3, dadurch gekennzeichnet, dass es ein 5,6-Dihydro-1,2,4,6-thiatriazin(5)on-1,1-dioxid gemäss Anspruch 1 enthält, nämlich 6-Methyl-3-methoxy-5,6-dihydro-1,2,4,6-thiatriazin(5)on-1,1-dioxid, 6-Methyl-3-äthoxy-5,6-dihydro-1,2,4,6-thiatriazin(5)on-1,1-dioxid, 6-Äthyl-3-methoxy-5,6-dihydro-1,2,4,6-thiatriazin-(5)on-1,1-dioxid oder 6-Äthyl-3-äthoxy-5,6-dihydro-1,2,4,6-thiatriazin(5)on-1,1-dioxid.

5. Herbizid gemäss Anspruch 3, enthaltend zusätzlich einen festen oder flüssigen Trägerstoff.

6. Verfahren zur Herstellung eines Herbizids gemäss Anspruch 5, dadurch gekennzeichnet, dass man einen festen oder flüssigen Trägerstoff vermischt mit einen 5,6-Dihydro-1,2,4,6-thiatriazin(5)-on-1,1-dioxid gemäss Anspruch 1.

7. Verfahren zur Bekämpfung unerwünschten Pflanzenwuchses, dadurch gekennzeichnet, dass man die Pflanzen oder den Boden behandelt mit einem 5,6-Dihydro-1,2,4,6-thiatriazin(5)on-1,1-dioxid gemäss Anspruch 1.

8. Verfahren zur Herstellung eines 5,6-Dihydro--1,2,4,6-thiatriazin(5)on-1,1-dioxid gemäss Anspruch 1, dadurch gekennzeichnet. das man einen N-Carbo-alkoxy-O-alkyl-isoharnstoff der Formel

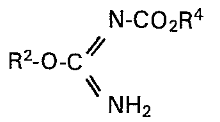

in der $R^2$ die vorgenannte Bedeutung besitzt und $R^4$ einen Alkylrest mit 1 bis 4 Kohlenstoffatomen bedeutet, mit einem Aminosulfonylhalogenid der Formel

$R^3$-$NHSO_2Y$

in der $R^3$ die vorgenannte Bedeutung besitzt und Y für Fluor oder Chlor steht, gegebenenfalls in Gegenwart eines Säureakzeptors und eines inerten organischen Lösungsmittels bei einer Temperatur im Bereich von −20 bis +80°C zu einem Sulfondiamid der Formel

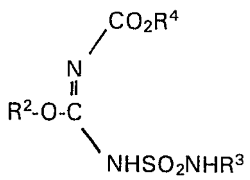

in der $R^2$, $R^3$ und $R^4$ die vorgenannten Bedeutungen besitzen, umsetzt und dieses in Gegenwart einer basischen Verbindung bei einer Temperatur im Bereich von 0 bis 100°C cyclisiert.

**Patentansprüche für den Vertragsstaat:** AT

1. Herbizid, enthaltend ein 5,6-Dihydro-1,2,4,6-thiatriazin(5)on-1,1-dioxid der Formel

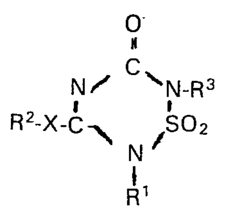

I

in der

$R^1$ Wasserstoff, ein Metallatom oder einen gegebenenfalls durch Alkyl ($C_1$-$C_{13}$) substituierten Ammoniumrest,

$R^2$ einen gesättigten oder ungesättigten geradkettigen aliphatischen Rest mit bis zu 10 Kohlenstoffatomen, einen cycloaliphatischen Rest mit 3 bis 7 Kohlenstoffatomen, einen verzweigten gesättigten oder ungesättigten aliphatischen Rest mit 3 bis 10 Kohlenstoffatomen, einen halogen-, methoxy- oder methylmercapto-substituierten aliphatischen Rest mit 2 bis 10 Kohlenstoffatomen, einen cyclohexoxy-substituierten aliphatischen Rest mit 4 bis 10 Kohlenstoffatomen, einen gegebenenfalls halogen-substituierten Benzylrest oder einen gegebenenfalls halogen-, tertiärbutyl- oder isopropyl-substituierten Phenylrest mit bis zu 10 Kohlenstoffatomen,

$R^3$ Wasserstoff, einen geradkettigen aliphatischen Rest mit bis zu 10 Kohlenstoffatomen, einen cycloaliphatischen Rest mit 3 bis 7 Kohlenstoffatomen, einen verzweigten aliphatischen Rest mit 3 bis 10 Kohlenstoffatomen oder einen halogen- oder methoxysubstituierten Alkylrest mit 2 bis 10 Kohlenstoffatomen und

X Sauerstoff bedeutet.

2. Herbizid gemäss Anspruch 1, dadurch gekennzeichnet, dass es ein 5,6-Dihydro-1,2,4,6-thiatriazin(5)on-1,1-dioxid enthält, nämlich 6-Methyl-3-methoxy-5,6-dihydro-1,2,4,6-thiatriazin(5)on-1,1-dioxid, 6-Methyl-3-äthoxy-5,6-dihydro-1,2,4,6-thiatriazin(5)on-1,1-dioxid, 6-Äthyl-3-methoxy-5,6-dihydro-1,2,4,6-thiatriazin(5)on-1,1-dioxid od. 6-Äthyl-3-äthoxy-5,6-dihydro-1,2,4,6-thiatriazin-(5)on-1,1-dioxid.

3. Herbizid gemäss Anspruch 1, enthaltend zusätzlich einen festen oder flüssigen Trägerstoff.

4. Verfahren zur Herstellung eines Herbizids gemäss Anspruch 3, dadurch gekennzeichnet, dass man einen festen oder flüssigen Trägerstoff vermischt mit einem 5,6-Dihydro-1,2,4,6-thiatriazin-(5)on-1,1-dioxid definiert wie Anspruch 1,

5. Verfahren zur Bekämpfung unerwünschten Pflanzenwuchses, dadurch gekennzeichnet, dass man die Pflanzen oder den Boden behandelt mit einem 5,6-Dihydro-1,2,4,6-thiatriazin(5)on-1,1-dioxid definiert wie im Anspruch 1.

6. Verfahren zur Herstellung eines 5,6-Dihydro-1,2,4,6-thiatriazin(5)on-1,1-dioxid definiert wie im Anspruch 1, dadurch gekennzeichnet, dass man einen N-Carbo-alkoxy-O-alkyl-isoharnstoff der Formel

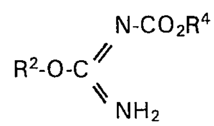

in der $R^2$ die vorgenannte Bedeutung besitzt und $R^4$ einen Alkylrest mit 1 bis 4 Kohlenstoffatomen bedeutet, mit einem Aminosulfonylhalogenid der Formel

$R^3$-$NHSO_2Y$

in der $R^3$ die vorgenannte Bedeutung besitzt und Y für Fluor oder Chlor steht, gegebenenfalls in Gegenwart eines Säureakzeptors und eines inerten organischen Lösungsmittels bei einer Temperatur im Bereich von −20 bis +80°C zu einem Sulfondiamid der Formel

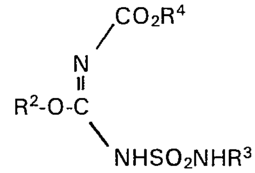

in der $R^2$, $R^3$ und $R^4$ die vorgenannten Bedeutungen besitzen, umsetzt und dieses in Gegenwart einer basischen Verbindung bei einer Temperatur im Bereich von 0 bis 100°C cyclisiert.

**Claims for the Contracting States:**
BE, CH, LI, DE, FR, GB, IT, NL, SE

1. A 5,6-dihydro-1,2,4,6-thiatriazin-5-one-1,1-dioxide of the formula

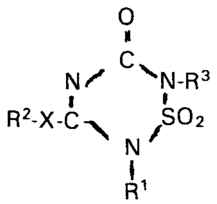

where
$R^1$ is hydrogen, a metal atom or an unsubstituted or $C_1$-$C_{13}$-alkyl-substituted ammonium radical,
$R^2$ is a saturated or unsaturated straight-chain aliphatic radical of up to 10 carbon atoms, a cycloaliphatic radical of 3 to 7 carbon atoms, a branched saturated or unsaturated aliphatic radical of 3 to 10 carbon atoms, a halogen-, methoxy- or methylmercapto-substituted aliphatic radical of 2 to 10 carbon atoms, a cyclohexoxy-substituted aliphatic radical of 4 to 10 carbon atoms, unsubstituted or halogen-substituted benzyl or unsubstituted or halogen-, tert.-butyl- or isopropyl-substituted phenyl of up to 10 carbon atoms,
$R^3$ is hydrogen, a straight-chain aliphatic radical of up to 10 carbon atoms, a cycloaliphatic radical of 3 to 7 carbon atoms, a branched aliphatic radical of 3 to 10 carbon atoms, or a halogen- or methoxy-substituted alkyl of 2 to 10 carbon atoms, and
X is oxygen.

2. A 5,6-dihydro-1,2,4,6-thiatriazin-5-one-1,1-dioxide as claimed in claim 1, namely 6-methyl-3-methoxy-5,6-dihydro-1,2,4,6-thiatriazin-5-one-1,1-dioxide, 6-methyl-3-ethoxy-5,6-dihydro-1,2,-4,6-thiatriazin-5-one-1,1-dioxide, 6-ethyl-3-methoxy-5,6-dihydro-1,2,4,6-thiatriazin-5-one-1,1-dioxide and 6-ethyl-3-ethoxy-5,6-dihydro-1,2,4,6-thiatriazin-5-one-1,1-dioxide.

3. A herbicide containing a 5,6-dihydro-1,2,4,6-thiatriazin-5-one-1,1-dioxide as claimed in claim 1.

4. A herbicide as claimed in claim 3, containing a 5,6-dihydro-1,2,4,6-thiatriazin-5-one-1,1-dioxide as claimed in claim 1, namely 6-methyl-3-methoxy-5,6-dihydro-1,2,4,6-thiatriazin-5-one-1,1-dioxide, 6-methyl-3-ethoxy-5,6-dihydro-1,2,4,6-thiatriazin-5-one-1,1-dioxide, 6-ethyl-3-methoxy-5,6-dihydro-1,2,4,6-thiatriazin-5-one-1,1-dioxide and 6-ethyl-3-ethoxy-5,6-dihydro-1,2,4,6-thiatriazin-5-one-1,1-dioxide.

5. A herbicide as claimed in claim 3, additionally containing a solid or liquid carrier.

6. A process for manufacturing a herbicide as claimed in claim 5, wherein a solid or liquid carrier is mixed with a 5,6-dihydro-1,2,4,6-thiatriazin-5-one-1,1-dioxide as claimed in claim 1.

7. A process for controlling the growth of unwanted plants, wherein the plants or the soil are treated with a 5,6-dihydro-1,2,4,6-thiatriazin-5-one-1,1-dioxide as claimed in claim 1.

8. A process for the manufacture of a 5,6-dihydro-1,2,4,6-thiatriazin-5-one-1,1-dioxide as claimed in claim 1, wherein an N-carboalkoxy-O-alkylisourea of the formula

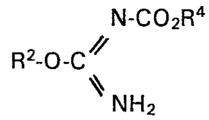

where $R^2$ has the above meanings and $R^4$ is alkyl of 1 to 4 carbon atoms, is reacted with an aminosulfonyl halide of the formula

$$R^3\text{-}NHSO_2Y$$

where $R^3$ has the above meanings and Y is fluorine or chlorine, in the presence or absence of an acid acceptor and of an inert organic solvent, of from −20 to +80°C, to give a sulfonediamide of the formula

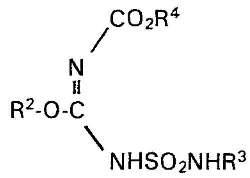

where $R^2$, $R^3$ and $R^4$ have the above meanings, and this compound is cyclized in the presence of a basic compound at from 0 to 100°C.

**Claims for the Contracting state:** AT

1. A herbicide containing a 5,6-dihydro-1,2,4,6-thiatriazin-5-one-1,1-dioxide of the formula

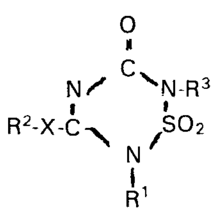 I

where
$R^1$ is hydrogen, a metal atom or an unsubstituted or $C_1$-$C_{13}$-alkyl-substituted ammonium radical,
$R^2$ is a saturated or unsaturated straight-chain aliphatic radical of up to 10 carbon atoms, a cycloaliphatic radical of 3 to 7 carbon atoms, a branched saturated or unsaturated aliphatic radical of 3 to 10 carbon atoms, a halogen-, methoxy- or methylmercapto-substituted aliphatic radical of 2 to 10 carbon atoms, a cyclohexoxy-substituted aliphatic radical of 4 to 10 carbon atoms, unsubstituted or halogen-substituted benzyl or unsubstituted or halogen-, tert.-butyl- or isopropyl-substituted phenyl of up to 10 carbon atoms,
$R^3$ is hydrogen, a straight-chain aliphatic radical of up to 10 carbon atoms, a cycloaliphatic radical of 3 to 7 carbon atoms, a branched aliphatic radical of 3 to 10 carbon atoms, or a halogen- or methoxy-substituted alkyl of 2 to 10 carbon atoms, and
X is oxygen.

2. A herbicide as claimed in claim 1, containing a 5,6-dihydro-1,2,4,6-thiatriazin-5-one-1,1-dioxide,

namely 6-methyl-3-methoxy-5,6-dihydro-1,2,4,6-thiatriazin-5-one-1,1-dioxide, 6-methy-3-ethoxy-5,6-dihydro-1,2,4,6-thiatriazin-5-one-1,1-dioxide, 6-ethyl-3-methoxy-5,6-dihydro-1,2,4,6-thiatriazin-5-one-1,1-dioxide and 6-ethyl-3-ethoxy-5,6-dihydro-1,2,4,6-thiatriazin-5-one-1,1-dioxide.

3. A herbicide as claimed in claim 1, additionally containing a solid or liquid carrier.

4. A process for manufacturing a herbicide as claimed in claim 3, wherein a solid or liquid carrier is mixed with a 5,6-dihydro-1,2,4,6-thiatriazin-5-one-1,1-dioxide as defined in claim 1.

5. A process for controlling the growth of unwanted plants, wherein the plants or the soil are treated with a 5,6-dihydro-1,2,4,6-thiatriazin-5-one-1,1-dioxide as defined in claim 1.

6. A process for the manufacture of a 5,6-dihydro-1,2,4,6-thiatriazin-5-one-1,1-dioxide as defined in claim 1, wherein an N-carboalkoxy-O-alkylisourea of the formula

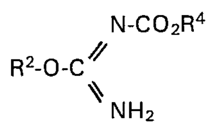

where $R^2$ has the above meanings and $R^4$ is alkyl of 1 to 4 carbon atoms, is reacted with an aminosulfonyl halide of the formula

$$R^3\text{-}NHSO_2Y$$

where $R^3$ has the above meanings and Y is fluorine or chlorine, in the presence or absence of an acid acceptor and of an inert organic solvent, of from −20 to +80°C, to give a sulfonediamide of the formula

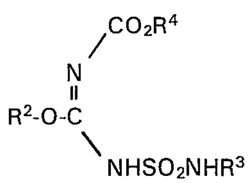

where $R^2$, $R^3$ and $R^4$ have the above meanings, and this compound is cyclized in the presence of a basic compound at from 0 to 100°C.

**Revendications pour les Etats contractants:**
BE, CH, LI, DE, FR, GB, IT, NL, SE

1. 5,6-dihydro-1,2,4,6-thiatriazin(5)one-1,1-dioxyde de formule

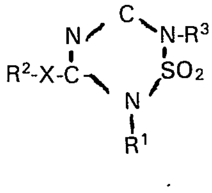

I

dans laquelle

$R^1$ représente hydrogène, un atome de métal ou un reste ammonium substitué éventuellement par alkyle ($C_1$-$C_{13}$),

$R^2$ représente un reste aliphatique, à chaîne droite, saturé ou non, ayant jusqu'à 10 atomes de carbone, un reste cycloaliphatique ayant 3 à 7 atomes de carbone, un reste aliphatique saturé ou non, ayant 3 à 10 atomes de carbone, un reste aliphatique substitué par halogène, méthoxy ou mercapto et ayant 2 à 10 atomes de carbone, un reste aliphatique, substitué par cyclohexoxy ayant 4 à 10 atomes de carbone, un reste benzyle, éventuellement substitué par halogène, ou un reste phényle ayant jusqu'à 10 atomes de carbone, éventuellement substitué par halogène, tert-butyle, ou isopropyle,

$R^3$ représente hydrogène, un reste aliphatique à chaîne droite ayant jusqu'à 10 atomes de carbone, un reste cycloaliphatique ayant 3 à 7 atomes de carbone, un reste aliphatique ramifié ayant 3 à 10 atomes de carbone, ou un reste alkyle ayant 2 à 10 atomes de carbone, substitué par halogène ou méthoxy

X représente oxygène.

2. 5,6-dihydro-1,2,4,6-thiatriazin(5)one-1,1-dioxyde selon la revendication 1, à savoir 6-méthyle-3-méthoxy-5,6-dihydro-1,2,4,6-thiatriazin(5)one-1,1-dioxyde, 6-méthyl-3-éthoxy-5,6-dihydro-1,2,-4,6-thiatriazin(5)one-1,1-dioxyde, 6-éthyl-3-méthoxy-5,6-dihydro-1,2,4,6-thiatriazin(5)one-1,1-dioxyde ou 6-éthyl-3-éthoxy-5,6-dihydro-1,2,4,6-thiatriazin(5)one-1,1-dioxyde.

3. Herbicide contenant un 5,6-dihydro-1,2,4,6-thiatriazin(5)one-1,1-dioxyde selon la revendication 1.

4. Herbicide selon la revendication 3, caractérisé par le fait qu'il contient un 5,6-dihydro-1,2,4,6-thiatriazin(5)one-1,1-dioxyde selon la revendication 1, à savoir 6-méthyl-3-méthoxy-5,6-dihydro-1,2,4,6-thiatriazin(5)one-1,1-dioxyde, 6-méthyl-3-éthoxy-5,6-dihydro-1,2,4,6-thiatriazin(5)one-1,1-dioxyde, 6-éthyl-3-méthoxy-5,6-dihydro-1,2,4,6-thiatriazin(5)one-1,1-dioxyde ou 6-éthyl-3-éthoxy-5,6-dihydro-1,2,4,6-thiatriazin(5)one-1,1-dioxyde.

5. Herbicide selon la revendication 3 contenant en outre un support solide ou liquide.

6. Procédé de préparation d'un herbicide selon la revendication 5, caractérisé par le fait qu'on mélange un support solide ou liquide avec un 5,6-dihydro-1,2,4,6-thiatriazin(5)one-1,1-dioxyde selon la revendication 1.

7. Procédé de lutte contre la croissance indésirable des plantes, caractérisé par le fait qu'on traite les plantes ou le sol avec un 5,6-dihydro-1,2,4,6-thiatriazin(5)one-1,1-dioxyde selon la revendication 1.

8. Procédé de préparation d'un 5,6-dihydro-1,2,4,6-thiatriazin(5)one-1,1-dioxyde selon la revendication 1, caractérisé par le fait qu'on transforme une N-carbo-alcoxy-O-alkyl-isourée de formule

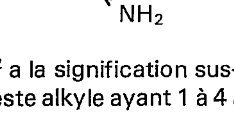

dans laquelle $R^2$ a la signification sus-indiquée et $R^4$ représente un reste alkyle ayant 1 à 4 atomes de carbone, par réaction avec un halogénure d'aminosulfonyle de formule

$R^3$-$NHSO_2Y$

dans laquelle $R^3$ a la signification sus-indiquée et Y représente fluor ou chlore, éventuellement en présence d'un accepteur d'acide et d'un solvant organique inerte, à une température de −20 à +80°C, pour obtenir un sulfondiamide de formule

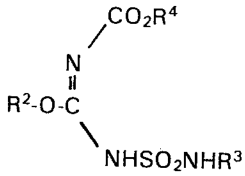

dans laquelle $R^2$, $R^3$ et $R^4$ ont les significations sus-indiquées, et on cyclise celui-ci en présence d'un composé basique à une température de 0 à 100°C.

**Revendications pour l'Etat contactant:** AT

1. Herbicide contenant un 5,6-dihydro-1,2,4,6-thiatriazin(5)one-1,1-dioxyde de formule

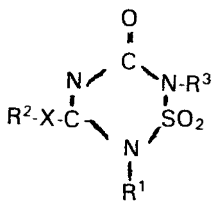

I

dans laquelle

$R^1$ représente hydrogène, un atome de métal ou un reste ammonium substitué éventuellement par alkyle ($C_1$-$C_{13}$),

$R^2$ représente un reste aliphatique, à chaîne droite, saturé ou non, ayant jusqu'à 10 atomes de carbone, un reste cycloaliphatique ayant 3 à 7 atomes de carbone, un reste aliphatique saturé ou non, ayant 3 à 10 atomes de carbone, un reste aliphatique substitué par halogène, méthoxy ou mercapto et ayant 2 à 10 atomes de carbone, un reste aliphatique, substitué par cyclohexoxy ayant 4 à 10 atomes de carbone, un reste benzyle, éventuellement substitué par halogène, ou un reste phényle ayant jusqu'à 10 atomes de carbone, éventuellement substitué par halogène, tert-butyle, ou isopropyle,

$R^3$ représente hydrogène, un reste aliphatique à chaîne droite ayant jusqu'à 10 atomes de carbone, un reste cycloaliphatique ayant 3 à 7 atomes de carbone, un reste aliphatique ramifié ayant 3 à 10 atomes de carbone, ou un reste alkyle ayant 2 à 10 ato-

X représente oxygène.

2. Herbicide selon la revendication 1, caractérisé par le fait qu'il contient un 5,6-dihydro-1,2,4,6-thiatriazin(5)one-1,1-dioxyde selon la revendication 1, à savoir 6-méthyl-3-méthoxy-5,6-dihydro-1,2,4,6-thiatriazin(5)one-1,1-dioxyde, 6-méthyl-3-éthoxy-5,6-dihydro-1,2,4,6-thiatriazin(5)one-1,1-dioxyde, 6-éthyl-3-méthoxy-5,6-dihydro-1,2,4,6-thiatriazin(5)one-1,1-dioxyde ou 6-éthyl-3-éthoxy-5,6-dihydro-1,2,4,6-thiatriazin(5)one-1,1-dioxyde.

3. Herbicide selon la revendication 1, contenant en outre un support solide ou liquide.

4. Procédé de préparation d'un herbicide selon la revendication 3, caractérisé par le fait qu'on mélange un support solide ou liquide avec un 5,6-dihydro-1,-2,4,6-thiatriazin(5)one-1,1-dioxyde défini comme dans la revendication 1.

5. Procédé de lutte contre la croissance indésirable des plantes, caractérisé par le fait qu'on traite les plantes ou le sol avec un 5,6-dihydro-1,2,4,6-thiatriazin(5)one-1,1-dioxyde défini comme dans la revendication 1.

6. Procédé de préparation d'un 5,6-dihydro-1,2,4,6-thiatriazin(5)one-1,1-dioxyde selon la revendication 1, caractérisé par le fait qu'on transforme une N-carbo-alcoxy-O-alkyl-isourée de formule

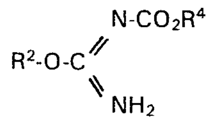

dans laquelle $R^2$ a la signification sus-indiquée et $R^4$ représente un reste alkyle ayant 1 à 4 atomes de carbone, par réaction avec un halogénure d'aminosulfonyle de formule

$R^3$-$NHSO_2Y$

dans laquelle $R^3$ a la signification sus-indiquée et Y représente fluor ou chlore, éventuellement en présence d'un accepteur d'acide et d'un solvant organique inerte, à une température de −20 à +80°C, pour obtenir un sulfondiamide de formule

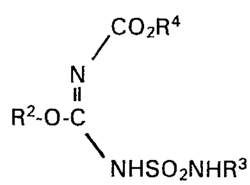

dans laquelle $R^2$, $R^3$ et $R^4$ ont les significations sus-indiquées, et on cyclise celui-ci en présence d'un composé basique à une température de 0 à 100°C.